# EUROPEAN PATENT SPECIFICATION

(11) **EP 2 957 552 B1**
(45) Date of publication and mention of the grant of the patent: **22.01.2020**
(21) Application number: 15172131.3
(22) Date of filing: 15.06.2015
(51) Int. Cl.: C07C 31/135, A61K 31/40, A61P 11/08

(54) **VILANTEROL FORMULATIONS**
VILANTEROLFORMULIERUNGEN
FORMULATIONS DE VILANTEROL

(30) Priority: 16.06.2014 TR 201406982; 17.06.2014 TR 201407010; 17.06.2014 TR 201407009
(43) Date of publication of application: 23.12.2015
(73) Proprietor: Arven Ilac Sanayi Ve Ticaret A.S., 34460 Istanbul (TR)
(72) Inventor: TÜRKYILMAZ, Ali, 34460 Istanbul (TR); CELIK, Devrim, 34460 Istanbul (TR); AKDAS, Özlem, 34460 Istanbul (TR)
(74) Representative: Mutlu, Aydin

(56) References cited:
- WO-A1-2010/097115
- Paolo Colombo, Daniela Traini, Francesca Buttini: "Section 5.1.2" In: inhalation drug delivery / techniques and products: 19 December 2012 (2012-12-19), John Wiley
- Javadzadeh et al: "Chapter 3, Section 6.4, page 47" In: "The role of carrier in dry powder inhaler", 2012

## Description

### Field of the invention

The present invention relates to a pharmaceutical formulation for inhalation comprising vilanterol as an active agent, a carrier and magnesium stearate in the treatment and prophylaxis of respiratory diseases such as asthma, chronic obstructive pulmonary disease (COPD) as well as to a process for producing this formulation.

### Background of the invention

β₂-adrenoceptor agonists are widely used in the treatment and prophylaxis of respiratory diseases associated with airflow obstruction such as asthma, chronic obstructive pulmonary disease (COPD). They provide rapid and effective symptom relief principally by opposing the bronchoconstriction induced by excitatory airway mediators. Therefore, β₂-adrenoceptor agonists improve lung function, symptoms of breathlessness and exercise limitation, health-related quality of life, and may also reduce the rate of exacerbations.

There are two types of β₂-adrenoceptor agonists: long-acting β₂-adrenoceptor agonist (LABA) and short-acting β₂-adrenoceptor agonist (SABA). Short-acting β₂- adrenoceptor agonists are used only as relief medication, whereas long-acting β₂- adrenoceptor agonists are used on a daily basis to improve lung function.

A LABA with a 24-hour duration of action could provide improvements in efficacy, compared with twice-daily LABAs, and the once-daily dosing regimen could help improve compliance. An important step in simplifying asthma and COPD management and improving compliance with prescribed therapy is to reduce the dose frequency to the minimum necessary to maintain disease control. Therefore, once-daily dose administration is an important strategy to improve compliance.

Vilanterol is a LABA with a 24-hour duration of action that is used for the preparation of a medicament in the prophylaxis and treatment of respiratory diseases such as asthma, chronic obstructive pulmonary diseases (COPD), respiratory tract infection and upper respiratory tract disease. It is also known with the chemical name of 4-{(1R)-2-[(6-{2-[(2,6-dichlorobenzyl)oxy]ethoxy}hexyl)amino]-1-hydroxyethyl}-2-(hydroxymethyl) phenol of fomula given below: 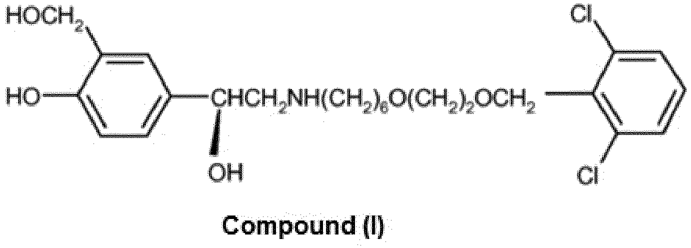 The compound (I) and pharmaceutically acceptable salts thereof, in particular the acetate, triphenylacetate, α-phenylcinnamate, 1 -naphthoate and (R)-mandelate salts, are specifically described in WO03/024439A1 as well as the preparation method of the compound I.

β₂-adrenoceptor agonists used in the treatment of respiratory diseases are administered through various routes (e.g. inhalation, oral, parenteral). However, the preferred route of administration of these drugs is inhalation, since they are directly delivered to the affected sites (airways) in high doses via this route, have a short onset time, and they lack or have minimal systemic side effects. The pharmaceutical formulations comprising said drugs are administered via nebulizers, pressurized metered dose inhalers (pMDIs), and dry powder inhalers (DPIs). In nebulizers and pMDIs, drugs are dispersed in water or propellant-solvent mixtures, and administered as aerosolized droplets of the dispersed systems. But in DPIs, drugs are administered as a powder after formulating them with inert carriers, including lactose, glucose, and mannitol. Compared to other pulmonary drug delivery systems, DPIs offer several advantages, including enhanced drug stability, greater accuracy in dosing, elimination of hand-to-mouth coordination, breath-actuated delivery, and consequently, an overall improvement in patient compliance.

Dry powder formulations, that are suitable to be used via DPI, must fulfil a number of demands. With the aim of fulfilling these demands, it would be highly advantageous to provide a formulation exhibiting good uniformity of distribution of the active ingredient, small drug dosage variation (in other words, adequate accuracy of the delivered

WO 2010/097115 A1 discloses dry powder vilanterol formulations. Vilanterol formulations comprise a carrier and a ternary agent which may be magnesium stearate. Sustained bronchodilation with a safety profile is indicated as an objective. This document is however silent on vilanterol formulations having good uniformity of distribution, adequate accuracy of the delivered dose and high flowability, improved aerosolisation performance.

doses), good flowability, adequate physical stability in the device before use, good aerosolisation performance in terms of emitted dose and fine particle fraction (FPF).

The effective inhalation performance of dry powder products is dependent on the drug formulation and the inhaler device. Dry powder formulations are usually prepared by mixing the micronized drug particles with larger carrier particles. Drug deposition in the lung is mainly controlled by its mass median aerodynamic diameter. Particles larger than 5 µm are mostly trapped by oropharyngeal deposition and incapable of reaching the lungs while smaller than 1 µm are mostly exhaled without deposition. Particles with mass median aerodynamic diameters between 1 µm and 5 µm are expected to efficiently deposit in the lung periphery. However, microfine powder particles have high surface energy and thus they are highly adhesive and cohesive. It is well known that the surface of the carrier particles is, indeed, not smooth but has asperities and clefts, which are high energy sites on which the highly adhesive and cohesive active particles are preferably attracted to and adhere more strongly. These forces which occur between the two ingredients in the powder mixtures turn out to be too high thus preventing the separation of the micronised drug particles from the surface of the carrier particles during inhalation. Therefore, the magnitude of adhesion and cohesion forces is very important to produce a stable formulation that is homogenous mixture with proper content uniformity and with no powder segregation during the processing, storing and transportation, but allows for easy separation between drug and carrier particles during inhalation.

On the other hand, strong adhesion and cohesion forces lead to poor flowability and to powder aggregation. The poor flowability is also detrimental to the fine particle fraction (respirable fraction) of the delivered dose being the active particles unable to leave the inhaler and remaining adhered to the interior of the inhaler or leaving the inhaler as large agglomerates; agglomerated particles, in turn, cannot reach the bronchiolar and alveolar sites of the lungs. The uncertainty as to the extent of agglomeration of the particles between each actuation of the inhaler and also between inhalers and different batches of particles, leads to poor dose reproducibility as well.

Additionally, the poor flowability makes the powder to be difficult to handle and impairs the mechanical filling of the powder into medicament carriers (e.g. capsules, blisters, resevoirs, etc) with high accuracy and in correct dosage.

The patient compliance is very essential for the successful management of the respiratory diseases, especially asthma and COPD. For improvement in patient compliance, the important step is to reduce the dose frequency to the minimum necessary to maintain the treatment and this results in simplifying the management of respiratory diseases, especially asthma and COPD. Therefore, the once-daily dose administration is an important strategy to improve patient compliance.

In dry powder formulations for inhalation, it is necessary to deliver the drug with 24-hour duration of action to the lungs in efficient amount for the treatment to guarantee the maintenance the effect of the drug during 24-hour duration for success of the once-daily administration of said formulation.

Vilanterol is a LABA with a 24-hour duration of action that is used for the preparation of a medicament in the prophylaxis and treatment of respiratory diseases such as asthma, chronic obstructive pulmonary diseases (COPD), respiratory tract infection and upper respiratory tract disease. It is aimed in the present invention to provide a pharmaceutical formulation in dry powder form comprising vilanterol that overcomes the aforementioned drawbacks relating to the dry powders to deliver vilanterol in efficient amount in each inhalation to guarantee the maintenance the effect of vilanterol during 24-hour duration for the once-daily administration of said formulation.

### The Detailed Description of the Invention

The present invention relates to a dry powder formulation comprising vilanterol in which fine balance of the adhesive and cohesive forces between the drug and carrier particles is provided so as to ensure increase in the delivery efficiency of vilanterol to the lungs.

Dry powder formulations are generally formulated as a powder mixture of coarse carrier particles and micronized drug particles with mass median aerodynamic particle diameters of 1-5 µm. Carrier particles are used to improve drug particle flowability, thus improving dosing accuracy and minimizing the dose variability observed with drug formulations alone while making them easier to handle during manufacturing operations. With the use of carrier particles, drug particles are emitted from capsules and devices more readily, hence, the inhalation efficiency increases.

Usually no more than a few micrograms of a drug is used for the inhalation therapy, so it is very difficult to deliver the drug completely to the lung. Therefore, the carrier is used to enable bulk, which improves the handling, dispensing, and metering of the drug. Consequently, the carrier forms an important component of the formulation and any change in the properties of the carrier particles has the potential to alter the drug deposition profile. Therefore, the design of the carrier particle in terms of particle size, shape and surface properties is important for the aerosolization efficiency of dry powder formulations.

In order to get drug delivery into the lungs from a DPI formulation, the drug particles have to detach from surface of the carrier particles and penetrate into the lungs. The adhesive and cohesive forces between contiguous particle surfaces affect the detachment of the drug particles from surface of the carrier particles, and thus aerosolisation of respirable particles in dry powder formulation. The different surface properties of the carrier resulted in different adhesive forces between the drug and the carrier, which was reflected in the lung deposition results. During inhalation, the adhesive forces that exist between drug and carrier particles have to be overcome in order to aerosolise drug particles. The magnitude of the attachment forces during inhalation relative to the adhesive forces in the mixture determines the obtained fine particle fraction (FPF). Consequently, optimising a dry powder inhalation system with respect to delivered fine particle dose requires careful balancing between both types of forces. The attachment forces have to be strong enough to maintain satisfactory blend homogeneity during handling, storage and transportation but weak enough to yield a high drug release from the carrier particles during inhalation.

In dry powder formulations, it is known that the inclusion of fine carrier resulted in decreasing adhesion between the drug particles and carrier surface, therefore requiring a small amount of energy to detach the drug particles leading to an increased aerosol deposition performance (aerosolisation performance). Because, the interactions between coarse and fine particles in the formation of mixtures which proposed that fine particles preferentially bind to areas of high energy (active sites) on the surface of the carrier, leading the drug particles to bind to areas with lower energy. Once aerosolised, the drug particles are more easily detached from the surface of the carrier, increasing the proportion of drug available for inhalation. Therefore, the carrier containing greater proportions of fine carrier give better aerosol deposition performance.

It is also known that the addition of low surface free energy materials such as magnesium stearate, leucine, lecithin to the carrier-based dry powder formulation increases the aerosolisation efficiencies of dry powder inhaler formulations, by decreasing the drug-excipient adhesion and thus facilitating the drug detachment upon device actuation. The magnesium stearate is preferably used, for this purpose, in the dry powder formulations. When magnesium stearate is used in the dry powder formulations, some of the active sites of carrier particles are occupied by magnesium stearate and the interparticulate forces (i.e. adhesive forces and cohesive forces) are balanced in the formulation, therefore not being too weak which would lead to the falling off the drug particles and forming agglomerates and not too strong which would not enable their detachment from the carrier surface. However, only some of the active sites are occupied by magnesium stearate, enabling some active sites to be occupied by the drug particles and therefore aerosolise upon actuation.

Accordingly, the main object of the present invention to provide dry powder formulations of vilanterol for inhalation in which the interparticulate forces between vilanterol particles and carrier particles are balanced for achieving an efficient inhalation of said formulation to guarantee the maintenance the effect of vilanterol during 24-hour duration for the once-daily administration of said formulation.

It has been surprisingly found that when the dry powder formulation for inhalation is formulated to comprise vilanterol or a pharmaceutically acceptable salt thereof, coarse carrier, fine carrier, and magnesium stearate in which the ratio of the volume median diameter of the fine carrier to the volume median diameter of the magnesium stearate is 1:1, the adhesive and cohesive forces between vilanterol and carrier particles are well-balanced and the dry powder formulation that exhibits good uniformity of distribution of vilanterol, small drug dosage variation (in other words, adequate accuracy of the delivered doses), good flowability, adequate stability in the device before use, good aerosolisation performance in terms of emitted dose and fine particle fraction (FPF) is provided.

The coarse carrier particles have a heterogeneous rough surface with clefts and asperities that enables a range of weak and strong adhesive interaction between the drug particles and the carrier surface and thus particle detachments occur over a wide range of energies. When magnesium stearat and fine carrier particles are used in such a way that the ratio of the volume median diameter of fine carrier particles to the volume median diameter of magnesium stearate is between 1:1 besides the coarse carrier particles in the dry powder formulation comprising vilanterol or a pharmaceutically acceptable salt, a synergistic effect is obtained in terms of interparticulate forces between vilanterol and carrier particles in comparison with the dry powder formulation of vilanterol or a pharmaceutically acceptable salt that contains fine carrier particles or magnesium stearate alone besides coarse carrier particles. Addition of fine carrier particles and magnesium stearate with defined volume median diameter improves the mass median aerodynamic performance of vilanterol in such a way that the number of free active sites with a wide range of energies on the coarse carrier surface was occupied by said fine carrier particles and magnesium stearate particles and vilanterol particles is more effectively delivered.

The present invention provides a dry powder formulation for inhalation comprising vilanterol or a pharmaceutically acceptable salt thereof, coarse carrier particles, fine carrier particles and magnesium stearate particles in which the ratio of the volume median diameter of fine carrier particles to the volume median diameter of magnesium stearate is 1:1.

In other words, this results in the interaction between vilanterol and coarse carrier particles to be optimized to produce the stable formulation that is homogenous mixture with proper content uniformity and with no powder segregation during the processing, storing and transportation, but allows for easy separation between vilanterol and carrier particles during inhalation. The vilanterol particles are detached from the carrier surface easily in dry powder formulations when exposed to an air flow of the patient during inhalation. Thus, the dry powder formulation of the present invention improves aerosolisation performance in terms of emitted dose and fine particle fraction (FPF) to be provided.

According to the present invention, "emitted dose (or delivered dose)" is the total amount of the active agent emitted from the inhaler device and hence available to the user. Fine particle fraction (FPF) is defined as the percentage of active agent (<5 µm in mass median aerodynamic diameter) which is deposited into respirable regions of the lung, divided by the total amount of active agent leaving the device. In order to achieve a good aerosolisation performance when dry powder formulations are developed, it is generally intended that the emitted dose is above 90% and the FPF is above 15%.

The volume median diameter (Dv₅₀ or Dv_{0.5}) is the median for a volume distribution in such a way that 50% of the volume of the particle diameter is less than the median and 50% of the volume of the particles diameter is more than the median.

The volume median diameter of the fine carrier, the volume median diameter of the coarse carrier and the volume median diameter of the magnesium stearate contained in the dry powder formulation according to the present invention is preferably measured by means of a laser diffraction method. More specifically, the volume median diameter of the fine carrier and the volume median diameter of the coarse carrier are measured using a dry dispersion method using air as a dispensing agent on a "Malvern Mastersizer 2000 Particle Size Analyzer". The volume median diameter of the magnesium stearate is also measured using a dry dispersion method using air as a dispensing agent on a "Malvern Mastersizer 2000 Particle Size Analyzer".

The dry powder formulation of the present invention has also a good flowability for inhaler filling and subsequently the fludisation of the formulation during device activation. This allows accurate metering of said dry powder formulation. Therefore, said formulation can be uniformly filled into blisters, capsules or reservoirs suitably used in dry powder inhalers, and thus, any dose inhaled by a patient from the respective blister, capsule, or reservoir during inhalation can be delivered with a high dose accuracy. Having said that, the dry powder formulation with good flow properties contributes to an almost complete discharge of the powder from the inhaler during inhalation.

The dry powder formulation of the invention comprises vilanterol as an active agent. Vilanterol can be in the form of free base or a pharmaceutically acceptable salt, solvate, or physiologically functional derivative thereof, preferably in the form of a salt, in the formulation. Pharmaceutically acceptable salts of vilanterol according to the invention include those formed with both organic and inorganic acids or bases. Pharmaceutical acceptable acid addition salts of vilanterol include those formed from hydrochloric, hydrobromic, sulphuric, citric, tartaric, phosphoric, lactic, pyruvic, acetic, trifluoroacetic, triphenylacetic, phenylacetic, substituted phenyl acetic e.g. methoxyphenyl acetic, sulphamic, sulphanilic, succinic, oxalic, fumaric, maleic, malic, glutamic, aspartic, oxaloacetic, methanesulphonic, ethanesulphonic, arylsulponic (for example p-toluenesulphonic, benzenesulphonic, naphthalenesulphonic or naphthalenedisulphonic), salicylic, glutaric, gluconic, tricarballylic, mandelic, cinnamic, substituted cinnamic (for example, methyl, methoxy, halo or phenyl substituted cinnamic, including 4-methyl and 4-methoxycinnamic acid and α-phenyl cinnamic acid), ascorbic, oleic, naphthoic, hydroxynaphthoic (for example 1-or 3-hydroxy-2-naphthoic), naphthaleneacrylic (for example naphthalene-2-acrylic), benzoic, 4-methoxybenzoic, 2or 4-hydroxybenzoic, 4-chlorobenzoic, 4-phenylbenzoic, bezeneacrylic (for example 1,4 benzenediacrylic) and isethionic acids. Pharmaceutical acceptable base salts of vilanterol include ammonium salts, alkali metal salts such as those of sodium and potassium, alkaline earth metal salts such as those of calcium and magnesium and salts with organic bases such as dicyclohexyl amine and N-methyl-D-glucamine.

In one embodiment of the present invention, vilanterol triphenylacetate is preferably used in as active agent in the dry powder formulation.

Vilanterol is preferably present in an amount of 0.05 - 2.5%, more preferably present in an amount of 0.05 -1.5%, most preferably present in an amount of 0.1 -1.0 % by weight of the total formulation wherein the weight of vilanterol is calculated as the free base.

Drug deposition in the lung is mainly controlled by its mass median aerodynamic diameter (MMAD). Particles larger than 5 µm are mostly trapped by oropharyngeal deposition and incapable of reaching the lungs while smaller than 1 µm are mostly exhaled without deposition. Particles with mass median aerodynamic diameters between 1 µm and 5 µm are expected to deposit in the lung periphery. Therefore, vilanterol with the mass median aerodynamic diameter between 1 µm and 5 µm is used as an active agent in the dry powder of the present invention.

As used herein, the terms "therapeutic agent", "active agent" or "drug" refer to a substance, as a chemical compound or complex, that has a measurable beneficial physiological effect on the body, such as a therapeutic effect in treatment and prophylaxis of a disease or disorder, when administered in an effective amount.

The phrase "effective amount" refers to that amount of a substance that produces some desired local or systemic effect at a reasonable benefit/risk ratio applicable to any treatment.

The term "mass median aerodynamic diameter" (MMAD) is a measure of the aerodynamic size of a dispersed aerosol particle. The aerodynamic diameter is used to describe an aerosolized particle in terms of its settling behavior, and is the diameter of a unit density sphere having the same settling velocity, generally in air, as the particle in question. The aerodynamic diameter encompasses particle shape, density, and physical size. MMAD refers to the midpoint or median of the aerodynamic particle size distribution of an aerosolized collection of particles determined by Andersen cascade impactor (ACI), Next Generation Impactor (NGI), or Marple Miller impactor at each of the common flow rates.

Most of the amount of the dry powder formulations is consisted of carrier particle and the carrier particles are used to improve drug particle flowability, thus improving dosing accuracy and minimizing the dose variability observed with drug formulations alone while making them easier to handle during manufacturing operations. With the use of carrier particles, drug particles are emitted from capsules and devices more readily, hence, the inhalation efficiency increases. Because of that, the selection of the carrier is very important for the aerosolisation performance of the dry powder formulation.

According to present invention, each of the fine carrier and the coarse carrier is a pharmaceutically acceptable carrier that is selected from the group comprising lactose, mannitol, glucose, trehalose, cellobiose, sorbitol, maltitol or a combination of two or more of them. Additionally, the type of the fine carrier can be the same as or different from the type of the coarse carrier. The fine carrier and coarse carrier may constitute a combination of mannitol and glucose, or mannitol and trehalose, or mannitol and sorbitol, or mannitol and cellobiose, or mannitol and maltitol, or lactose and mannitol, or lactose and glucose, or lactose and trehalose, or lactose and sorbitol, or lactose and cellobiose, lactose and maltitol. According to present invention, lactose is preferably used as both of the fine carrier and the coarse carrier. In one embodiment of the present invention, lactose is anyhdrous lactose or lactose monohydrate.

According to the invention, "pharmaceutically acceptable" refers to the properties and/or substances which are acceptable to the patient from a pharmacological-toxicological point of view and to the manufacturing pharmaceutical formulation.

According to present invention, the volume median diameter of the magnesium stearate is between 1 µm and 120 µm, for example 5 µm, 10 µm, 15 µm, 20 µm, 25 µm, 30 µm, 35 µm, 40 µm, 45 µm, 60 µm, 75 µm, 80 µm, 85 µm, 90 µm, 100 µm, 110 µm; preferably between 1 µm and 50 µm, for example 4 µm, 8 µm, 12 µm, 16 µm, 20 µm, 24 µm, 28 µm, 32 µm, 36 µm, 40 µm, 44 µm, 48 µm; more preferably between 1 µm and 25 µm, for example 3 µm, 6 µm, 9 µm, 11 µm, 14 µm, 18 µm, 21 µm, 23 µm; most preferably 1 µm and 15 µm, for example 2 µm, 4 µm, 7 µm, 8 µm; the volume median diameter of the fine carrier, preferably fine lactose, is between 1 µm and 20 µm, for example 2 µm, 3 µm, 4 µm, 5 µm, 7 µm, 9 µm, 11 µm, 13 µm, 15 µm, 17 µm, 18 µm, 19 µm; preferably between 1 µm and 15 µm for example 1.5 µm, 2.5 µm, 3.5 µm, 4.5 µm, 5.5 µm, 6.5 µm, 7.5 µm, 8.5 µm, 9.5 µm, 10.5 µm, 11.5 µm, 12.5 µm, 13.5 µm, 14.5 µm; more preferably between 1 µm and 10 µm, for example 1.4 µm, 2.4 µm, 3.4 µm, 4.4 µm, 5.4 µm, 6.4 µm, 7.4 µm, 8.4 µm, 9.4 µm; most preferably 1 µm and 6 µm, for example 1.7 µm, 2.7 µm, 3.7 µm, 4.7 µm, 5.7 µm.

The amount of the coarse carrier particles is much more than the amount of fine carrier particle and magnesium stearate in the dry powder formulation of the present invention. Therefore, the particle size of the coarse carrier particles is also important for delivery performance of vilanterol in terms of fine particle fraction. The volume median diameter of the coarse carrier, preferably coarse lactose, used in the dry powder formulation of the present invention, is between 30 µm and 250 µm, for example 35 µm, 40 µm, 45 µm, 50 µm, 55 µm, 60 µm, 65 µm, 70 µm, 75 µm, 80 µm, 85 µm, 90 µm, 95 µm, 100 µm, 105 µm, 110 µm, 115 µm, 120 µm, 125 µm, 130 µm, 135 µm, 140 µm, 145 µm, 150 µm, 155 µm, 160 µm, 165 µm, 170 µm, 175 µm, 180 µm, 185 µm, 190 µm, 195 µm, 200 µm, 205 µm, 210 µm, 215 µm, 220 µm, 225 µm, 230 µm, 235 µm, 240 µm, 245 µm; preferably between 40 µm and 225 µm, for example 43 µm, 48 µm, 57 µm, 64 µm, 76 µm, 82 µm, 93 µm, 106 µm, 119 µm, 121 µm, 133 µm, 142 µm, 151 µm, 165 µm, 173 µm, 186 µm, 192 µm, 203 µm, 207 µm, 211 µm, 216 µm, 218 µm, 222 µm; more preferably between 45 µm and 215 µm, for example 47 µm, 52 µm, 58 µm, 66 µm, 72 µm, 83 µm, 91 µm, 103 µm, 117 µm, 125 µm, 132 µm, 138 µm, 143 µm, 149 µm, 154 µm, 159 µm, 162 µm, 168 µm, 174 µm, 179 µm, 183 µm, 188 µm, 192 µm, 197 µm, 206 µm, 209 µm, 213 µm; most preferably 50 µm and 200 µm, for example 53 µm, 59 µm, 64 µm, 73 µm, 77 µm, 81 µm, 83 µm, 86 µm, 89 µm, 92 µm, 97 µm, 99 µm, 101 µm, 106 µm, 112 µm, 114 µm, 118 µm, 121 µm, 133 µm, 146 µm, 151 µm, 156 µm, 161 µm, 167 µm, 177 µm, 179 µm, 184 µm, 189 µm, 194 µm, 199 µm.

Moisture uptake can directly affect the flowability of the powders and the force to detach the micronized particles from the carrier surface. Use for magnesium stearate in the formulation of the present invention, helps to minimize the influence of penetrating moisture during the storage of said formulation and results in said formulation to be more stable against the moisture. Thus, the quality of the pharmaceutical formulation remains considerably better than conventional formulations which are free of magnesium stearate even on storage under extreme conditions of temperature and humidity. Therefore, use of magnesium stearate improves the moisture resistance of the dry powder formulations.

However, magnesium stearate is poorly water soluble, its presence in such amount may rise some concerns as to a potential irritation or toxicity of this excipient, part of which can be inhaled by the patient together with the active ingredient. Therefore, it is important to determine the optimum concentration of the magnesium stearate that enables eliminating or minimizing potential irritation or toxicity of this excipient while getting balanced interparticulate forces (i.e. between the drug particles and the carrier surface which will enable maximum aerosolisation deposition, and minimizing the influence of penetrating moisture during the storage of the formulation. According to the present invention, the optimum amount of magnesium stearate is found as less than 1.0% by weight based on the total amount of the dry powder formulation to achieve aforementioned effects at the same time. The preferred amount of the magnesium stearate contained in the formulation of the present invention is between 0.01% and 0.80%, for example 0.015%, 0.025%, 0.035%, 0.045%, 0.055%, 0.065%, 0.075%, 0.085%, 0.095%, 0.15%, 0.25%, 0.35%, 0.45%, 0.55%, 0.65%, 0.75%; more preferred amount of the magnesium stearate contained in the formulation of the present invention is between 0.02% and 0.50%, for example 0.04%, 0.06%, 0.08%, 0.12%, 0.16%, 0.18%, 0.22%, 0.24%, 0.26%, 0.28%, 0.32%, 0.34%, 0.36%, 0.38%, 0.42%, 0.44%, 0.46%, 0.48%; most preferred amount of the magnesium stearate contained in the formulation of the present invention is between 0.05% and 0.25%, for example 0.080%, 0.13%, 0.15%, 0.16%, 0.17%, 0.19%, 0.21%, 0.22%, 0.23%, by weight based on the total amount of the dry powder formulation.

The amount of fine carrier particles also play a key role in drug deposition. The concentration of the fine carrier particles in the formulation has also an effect in the aerosolisation performance of the formulation. According to present invention, the amount of the fine carrier particles, preferably fine lactose particles, is less than 25%, preferably between 1% and 20, more preferably between 1% and 15%; most preferably between 1% and 12%, for example 2%, 3%, 4%, 5%, 6%, 7%, 8%, 9%, 10%, by weight based on the total amount of the dry powder formulation of the present invention.

The dry powder formulation of the present invention is used in the prophylaxis and treatment of clinical conditions for which a selective β₂-adrenoreceptor agonist is indicated. Such conditions include diseases associated with reversible airways obstruction such as asthma, chronic obstructive pulmonary diseases (COPD) (e. g. chronic and wheezy bronchitis, emphysema), respiratory tract infection and upper respiratory tract disease (e.g. rhinitis, including seasonal and allergic rhinitis).

According to the present invention, the dry powder formulations can be delivered by any suitable inhalation device that is adapted to administer a controlled amount of such a pharmaceutical formulation in dry powder form to a patient. Suitable inhalation devices may rely upon the aerosolisation energy of the patient's own breath to expel and disperse the dry powder dose. Alternatively, this energy may be provided by an energy source independent of the patient's inhalation effort, such as by impellers, patient/device created pressurised gas sources or physically (e. g. compressed gas) or chemically stored energy sources. Suitable inhalation devices can also be of the reservoir type i. e. where the dose is withdrawn from a storage vessel using a suitably designed dosing device or alternatively, inhalation devices that release drug from pre-metered units e. g. blisters, cartridges or capsules.

According to the present invention, the total amount of the dry powder formulation per unit-pack (blister, cartridge, capsule, etc) is between 3 mg and 25 mg, preferably 5 mg and 15 mg.

There are various various types of dry powder inhalers, for example, reservoir dry powder inhalers, unit-dose dry powder inhalers, pre-metered multi-dose dry powder inhalers, nasal inhalers or insufflators. The formulation of the invention may be presented in unit dosage form. Dry powder formulations for topical delivery to the lung by inhalation may, for example, be presented in capsules and cartridges of for example gelatin, or blisters of for example laminated aluminium foil, for use in an inhaler or insufflator. Each capsule, cartridge or blister may contain between 1 µg and 100 µg, preferably between 2 µg and 75 µg, more preferably 5 µg and 50 µg of vilanterol as free base.

According to the present invention, the dry powder formulation comprising vilanterol is in a for suitable for administration by oral and nasal inhalation.

Packaging of the formulation may be suitable for unit dose or multi-dose delivery. In one embodiment, the formulation suitable for inhaled administration may be incorporated into a plurality of sealed dose containers provided on medicament pack(s) (e.g. blister) mounted inside a suitable inhalation device. The containers may be rupturable, peelable or otherwise openable one-at-a-time and the doses of the dry powder composition administered by inhalation on a mouthpiece of the inhalation device, as known in the art. The medicament pack may take a number of different forms, for instance a disk-shape or an elongate strip. Representative inhalation devices are the Diskhaler® and Diskus® devices, marketed by GlaxoSmithKline. The Diskus® inhalation device is, for example, described in GB 2242134A. A dry powder inhalable composition, may also be provided as a bulk reservoir in an inhalation device, the device then being provided with a metering mechanism for metering a dose of the composition from the reservoir to an inhalation channel where the metered dose is able to be inhaled by a patient inhaling at a mouthpiece of the device. Exemplary marketed devices of this type are Turbuhaler® of AstraZeneca, Twisthaler® of Schering and Clickhaler® of Innovata.

A further delivery method for a dry powder inhalable composition is for metered doses of the composition to be provided in capsules (one dose per capsule) which are then loaded into an inhalation device, typically by the patient on demand. The device has means to rupture, pierce or otherwise open the capsule so that the dose is able to be entrained into the patient's lung when they inhale at the device mouthpiece. As marketed examples of such devices there may be mentioned Rotahaler® of GlaxoSmithKline and Handihaler® of Boehringer Ingelheim.

The dry powder pharmaceutical composition of vilanterol or a pharmaceutically acceptable salt thereof in accordance with this invention can be prepared using standard methods. Vilanterol or a pharmaceutically acceptable salt, the fine carrier, the coarse carrier and magnesium stearate can be sequentially or simultaneously mixed using any suitable blending apparatus, such as high shear mixer (for example a QMM, PMA or TRV series mixer) or a low shear tumbling mixer (a Turbula mixer). The particular components of the formulation can be admixed in any order. Pre-mixing of particular components may be found to be advantageous in certain circumstances. Before mixing process, if it is necessary, any of the component of the formulation can be micronized to obtain the component with the desired particle size. The progress of the mixing process can be monitored by carrying out content uniformity determinations. For example, the mixing apparatus may be stopped, materials removed using a sample thief and then analysed for homogeneity by High Performance Liquid Chromatography (HPLC).

The preferred process for the preparation of the dry powder formulation for inhalation of the present invention comprises:
a) the total amount of the coarse carrier is divided into five fractions and the first fraction of the coarse carrier is put into a mixing vessel and it is mixed for a period of time to cover the inside of the wall of the mixing vessel,
b) then, the fine carrier, the magnesium stearate and vilanterol or a pharmaceutically acceptable salt thereof are completely added onto the first fraction of the carrier in step a) and they are mixed for a period of time (Premix A),
c) second, third and fourth fraction of the coarse carrier are added onto the Premix A respectively (Premix B); after every addition of one fraction of the coarse carrier to the mixture, they are mixed for a period of time,
d) finally, last fraction of the coarse carrier is added onto the Premix B and they are mixed for a period of time to obtain the dry powder formulation of the invention.

In one embodiment of the present invention, vilanterol triphenylacetate salt is used for the preparation of the dry powder formulation.

In another embodiment of the present invention, each of the fine carrier and coarse carrier, that are used for the preparation of the formulation of the invention, is a pharmaceutically acceptable carrier that is selected from the group comprising lactose, mannitol, glucose, trehalose, cellobiose, sorbitol, maltitol or a combination of two or more of them. Additionally, the type of the fine carrier can be same with or different from the type of the coarse carrier. The fine carrier and coarse carrier may constitute a combination of mannitol and glucose, or mannitol and trehalose, or mannitol and sorbitol, or mannitol and cellobiose, or mannitol and maltitol, or lactose and mannitol, or lactose and glucose, or lactose and trehalose, or lactose and sorbitol, or lactose and cellobiose, lactose and maltitol. According to present invention, lactose is preferably used as both of the fine carrier and the coarse carrier. Lactose used for the preparation of the dry powder formulation is anyhdrous lactose or lactose monohydrate.

Therefore, the more preferred process for the preparation of the dry powder formulation for inhalation comprises:
a) the total amount of the coarse lactose is divided into five fractions and the first fraction of the coarse lactose is put into a mixing vessel and it is mixed for a period of time to cover the inside of the wall of the mixing vessel,
b) then, the fine lactose, the magnesium stearate and vilanterol triphenylacetate are completely added onto the first fraction of the lactose in step a) and they are mixed for a period of time (Premix A),
c) second, third and fourth fraction of the coarse lactose are added onto the Premix A respectively (Premix B); after every addition of one fraction of the coarse lactose to the mixture, they are mixed for a period of time,
d) finally, last fraction of the coarse lactose is added onto the Premix B and they are mixed for a period of time to obtain the dry powder formulation of the invention.

In one embodiment of the present invention, in the preparation of the dry powder formulation, the Premix B is preferably sieved after step c). Then, the last fraction of the lactose is preferably added, through the same sieve that is used to sieve the Premix B, onto the Premix B to remove the remains attached to the sieve.

According to the present invention, the pharmaceutically acceptable carrier, preferably lactose, can be divided into five equal fractions or into five fractions in any proportion.

According to the present invention, vilanterol or a pharmaceutically acceptable salt thereof can be used in combination with one or more other therapeutic agents that is selected from a group comprising anti-inflammatory agents, anticholinergic agents (particularly a muscarinic (M₁, M₂, or M₃) receptor antagonist), other β₂-adrenoreceptor agonists, antiinfective agents (e.g. antibiotics, antivirals), or antihistamines for the preparation of the dry powder formulation. The invention thus provides, in a further embodiment, a combination comprising vilanterol or a pharmaceutically acceptable salt, solvate or physiologically functional derivative thereof, preferably vilanterol triphenylacetate, together with one or more other therapeutical active agents that is selected from a group comprising an anti-inflammatory agent (e.g. a corticosteroid or an NSAID), an anticholinergic agent, another β₂- adrenoreceptor agonist, an antiinfective agent (e. g. an antibiotic or an antiviral), or an antihistamine. Preferred are combinations comprising vilanterol or a pharmaceutically acceptable salt, solvate or physiologically functional derivative thereof, preferably vilanterol triphenylacetate, together with a corticosteroid (such as mometasone, fluticasone, budesonide) and/or an anticholinergic (such as tiotropium, oxitropium, glycopyrronium, ipratropium, aclidinium) and/or a PDE-4 inhibitor (such as roflumilast, rolipram, ibudilast, cilomilast).

The other therapeutic ingredient (s) may be used in the form of salts, (e. g. as alkali metal or amine salts or as acid addition salts), or prodrugs, or as esters (e. g. lower alkyl esters), or as solvates (e. g. hydrates). It will be clear also that where appropriate, the therapeutic ingredients may be used in optically pure form.

Vilanterol or a pharmaceutically acceptable salt thereof, preferably vilanterol triphenylacetate, and one or more other therapeutic agent(s) may be administered separately, sequentially or simultaneously in separate or combined pharmaceutical formulations in dry powder form. Vilanterol or a pharmaceutically acceptable salt thereof, preferably vilanterol triphenylacetate, and said other therapeutic agent(s) may be formulated separately and presented in separate packs or devices, or said individually formulated components may be present in a single pack or device. Where appropriate, the individual therapeutic agents may be admixed within the same formulation, and presented as a fixed pharmaceutical combination or formulated as a seperate formulation including pharmaceutically acceptable carriers and/or additives.

In one embodiment, vilanterol or a pharmaceutically acceptable salt thereof, preferably vilanterol triphenylacetate, may be used in combination with 6α, 9α-difluoro- 17α-[(2-furanylcarbonyl)oxy]-11β-hydroxy-16a-methyl-3-oxo-androsta-1,4-diene-17β-carbothioic acid S-fluoromethyl ester (fluticasone furoate) for the preparation of a pharmaceutical product for the treatment and prophylaxis of inflammatory or respiratory tract diseases.

Fluticasone furoate has also been the subject of extensive studies in animal models and humans and has been found to be a long acting inhaled glucocorticosteroid which has potential for once-daily administration to the lungs.

Fluticasone furoate is considered to have potential in the treatment of respiratory tract disease such as chronic obstructive pulmonary disease, chronic bronchitis, asthma, chronic respiratory obstruction, pulmonary fibrosis, pulmonary emphysema and allergic rhinitis.

For use according to the present invention, fluticasone furoate may be administered by inhalation at a dose of from about 25 µm to about 750 µm daily, and if necessary in divided doses. Thus, the daily dose of vilanterol may be at a dose of from about 1 µm to about 100 µm daily as free base, and if necessary in divided doses. In general, fluticasone furoate will be administered as a once-daily dose. fluticasone furoate may be provided in unit dose form, for example as described for vilanterol. Each unit dose of fluticasone furoate may contain between 25 µm and 750 µm of fluticasone furoate. The active agents (or therapeutic agents or drugs) used in the dry powder formulation of the invention preferably have a mass median aerodynamic diameter (MMAD) between 1 µm and 5 µm.

In another embodiment of the present invention, the present invention provides a pharmaceutical product comprising the dry powder formulation for inhalation of the present invention in combination with a dry powder formulation for inhalation comprising one or more other therapeutic agent(s), preferably 6α, 9α-difluoro- 17α-[(2-furanylcarbonyl)oxy]-11β-hydroxy-16α-methyl-3-oxo-androsta-1,4-diene-17β-carbothioic acid S-fluoromethyl ester (fluticasone furoate) and pharmaceutically acceptable carrier and/or additive.

In one embodiment said combination may be presented in the form of a pack comprising a dry powder formulation for inhalation comprising vilanterol or a pharmaceutically acceptable salt thereof, preferably vilanterol triphenylacetate, of the present invention and a separate dry powder formulation for inhalation of 6α, 9α-difluoro- 17α-[(2-furanylcarbonyl)oxy]-11β-hydroxy-16α-methyl-3-oxo-androsta-1,4-diene-17β-carbothioic acid S-fluoromethyl ester (fluticasone furoate).

Said pack may comprise two separate inhaler devices, containing respectively the separate formulations of vilanterol or a pharmaceutically acceptable salt thereof, preferably vilanterol triphenylacetate, and fluticasone furoate. Inhaler devices for delivery of fluticasone furoate include those described hereinabove for delivery of vilanterol.

Said pack may also comprise a delivery device which permits separate containment of the dry powder formulation of vilanterol or a pharmaceutically acceptable salt thereof, preferably vilanterol triphenylacetate, and the dry powder formulation of fluticasone furoate. Thus, for example, the individual compounds of the combination are administrable simultaneously but are stored separately, e.g. in separate pharmaceutical compositions.

In one embodiment, a delivery device permitting separate containment of actives is an inhaler device having two medicament packs in peelable blister strip form, each pack containing pre-metered doses in blister pockets arranged along its length. Said device has an internal indexing mechanism which, each time the device is actuated, peels opens a pocket of each strip and positions the packs so that each newly exposed dose of each pack is adjacent a manifold which communicates with a mouthpiece of the device. When the patient inhales at the mouthpiece, each dose is simultaneously drawn out of its associated pocket into the manifold and entrained via the mouthpiece into the patient's respiratory tract. Thus, each time the device is used, the patient is administered a combination therapy consisting of a dose from each medicament pack.

The present invention further provides a pharmaceutical product comprising a combination of vilanterol or a pharmaceutically acceptable salt thereof, preferably vilanterol triphenylacetate, and fluticasone propionate wherein at least vilanterol or a pharmaceutically acceptable salt thereof is formulated with magnesium stearate. Fluticasone furoate is also formulated with carrier, preferably fine and coarse carrier, optionally with magnesium stearate.

In another embodiment of the present invention, the present invention provides a pharmaceutical product comprising as separate formulation:
(a) a dry powder formulation comprising vilanterol or a pharmaceutically acceptable salt thereof of the present invention, and
(b) a dry powder formulation for inhalation comprising 6α, 9α-difluoro- 17α-[(2-furanylcarbonyl)oxy]-11β-hydroxy-16α-methyl-3-oxo-androsta-1,4-diene-17β-carbothioic acid S-fluoromethyl ester (fluticasone furoate), pharmaceutically acceptable carrier and/ or additive.

Vilanterol triphenylacetate salt is preferably used as the active agent in the dry powder formulation (a) of above mentioned pharmaceutical product.

According to the present invention, the pharmaceutically acceptable carrier used in the dry powder formulation of fluticasone furoat (b) is selected from the group comprising lactose, mannitol, glucose, trehalose, cellobiose, sorbitol, maltitol or a combination of two or more of them, for example a combination of mannitol and glucose, or mannitol and trehalose, or mannitol and sorbitol, or mannitol and cellobiose, or mannitol and maltitol, or lactose and mannitol, or lactose and glucose, or lactose and trehalose, or lactose and sorbitol, or lactose and cellobiose, lactose and maltitol. Lactose is preferably used as a pharmaceutically acceptable carrier in the dry powder formulation of fluticasone furoat. In one embodiment of the present invention, lactose is anyhdrous lactose or lactose monohydrate. On the other hand, if the dry powder formulation of fluticasone furoat comprises an additive, the additive is selected from a group comprising magnesium stearate, stearic acid, sodium lauryl sulphate, sodium stearyl fumarate, stearyl alcohol and sodium benzoate. Preferably, the magnesium stearate is used as the additive in said formulation.

Another embodiment of the disclosure is a method for the treatment and prophylaxis of inflammatory or respiratory tract diseases, which method comprises administering either sequentially or simultaneously, to a patient in need thereof, a pharmaceutical product comprising the dry powder formulation of vilanterol or a pharmaceutically acceptable salt thereof, preferably vilanterol triphenylacetate, and fluticasone furoate as defined above.

In one embodiment of the invention the respiratory disease is selected from the group consisting of chronic obstructive pulmonary disease, chronic bronchitis, asthma, chronic respiratory obstruction, pulmonary fibrosis, pulmonary emphysema and allergic rhinitis.

In another embodiment of the invention the pharmaceutical product may be used for the treatment of respiratory disease, and more specifically the treatment of asthma and/or chronic obstructive pulmonary disease (COPD), by simultaneous or successive administration of vilanterol or a pharmaceutically acceptable salt thereof and fluticasone furoate.

The present invention is explained in detail in the following examples. These examples are not limiting the scope of the present invention and are to be considered under the light of the foregoing detailed disclosure.

### Example

| **Formulation No** | **Vilanterol (as free base)** | **Coarse lactose** | **Fine lactose** | **Mg Stearate** | **VMD_{FLt}:VMD_{MgSt}** | **Total amount** |
|---|---|---|---|---|---|---|
| 1 | 0.10% - 1.5% | 85% - 98% | 1% - 15% | 0.05% - 0.25% | 1:1 | 3 - 25 mg |
| 2 | 0.10% - 1.5% | 85% - 98% | 1% - 15% | 0.05% - 0.25% | 3:1 | 3 - 25 mg |
| 3 | 0.10% - 1.5% | 85% - 98% | 1% - 15% | 0.05% - 0.25% | 1:10 | 3 - 25 mg |
| 4 | 0.10% - 1.5% | 85% - 98% | 1% - 15% | 0.05% - 0.25% | 5:1 | 3 - 25 mg |
| 5 | 0.10% - 1.5% | 85% - 98% | 1% - 15% | 0.05% - 0.25% | 1:5 | 3 - 25 mg |

| | | | | | | |
|---|---|---|---|---|---|---|
| *In the table, VMD_{FLt} is abbreviation of the volume median diameter of the fine lactose, VMD_{Mgst} is abbreviation of the volume median diameter of the magnesium stearat.* *VMD_{FLt}:VMD_{MgSt} is abbreviation of the ratio of the volume median diameter of fine lactose to the volume median diameter of magnesium stearat The percentage amount range of each component (showed in the table) is calculated based on the total amount of the total weight of the formulation.* | | | | | | |

### Preparation of Formulation-I

For the preparation of Formulation-1 in the table, initially the components of the Formulation-1 is weighted to the amount falling within the range that is showed in the table for each component. If it is necessary, any of the components of the formulation is micronized in a microniser (e.g. air-jet mill micronizer) to obtained said component with desired volume median diameter defined in the description before the mixing process. For the preparation of Formulation-1, the total amount of the coarse lactose is divided into five fractions and the first fraction of the coarse lactose is put into a mixing vessel and it is mixed for a period of time to cover the inside of the wall of the mixing vessel. Then, the fine lactose, the magnesium stearate and vilanterol triphenylacetate are completely added onto the first fraction of the lactose in step a) and they are mixed for a period of time (Premix A). Second, third and fourth fraction of the coarse lactose are added onto the Premix A respectively (Premix B); after every addition of one fraction of the coarse lactose to the mixture, they are mixed for a period of time. Finally, last fraction of the coarse lactose is added onto the Premix B and they are mixed for a period of time to obtain the dry powder formulation (Formulation-1). The obtained Formulation-1 is the dry powder formulation for inhalation in which the ratio of the volume median diameter of the fine lactose to the volume median diameter of the magnesium stearate (VMD_{FLt}:VMD_{MgSt}) is 1:1. Each of the mixing processes during the preparation of the dry powder formulation is performed using a high shear mixer or a low shear tumbling mixer whichever is appropriate.

Formula-2, Formula-3, Formula-4 and Formula-5 given in the table were prepared using the above-described procedure for the preparation of Formula-1.

## Claims

1. A dry powder formulation for inhalation comprising:
- vilanterol or a pharmaceutically acceptable salt thereof,
- coarse carrier,
- fine carrier, and
- magnesium stearate,
**characterized in that** the ratio of the volume median diameter of the fine carrier to the volume median diameter of the magnesium stearate is 1:1 and the volume median diameter of the magnesium stearate is between 1µm and 120 µm.

2. The dry powder formulation for inhalation according to claim 1, wherein the pharmaceutically acceptable salt of vilanterol is triphenylacetate salt.

3. The dry powder formulation for inhalation according to claim 1, wherein the fine carrier and the coarse carrier are selected from the group comprising lactose, mannitol, glucose, trehalose, cellobiose, sorbitol, maltitol or a combination of two or more of them.

4. The dry powder formulation for inhalation according to claim 3, wherein the type of the fine carrier is the same as or different from the type of the coarse carrier.

5. The dry powder formulation for inhalation according to claim 4, wherein both of the fine carrier and the coarse carrier is lactose.

6. The dry powder formulation for inhalation according to claim 1, wherein the amount of the magnesium stearate is less than 1.0% by weight based on the total amount of the dry powder formulation.

7. The dry powder formulation for inhalation according to any one of the preceding claims, wherein the amount of the fine carrier particles is less than 25% by weight based on the total amount of the dry powder formulation.

8. A process for the preparation of the dry powder formulation for inhalation according to any one of the preceding claims comprising:
a) the total amount of the coarse lactose is divided into five fractions and the first fraction of the coarse lactose is put into a mixing vessel and it is mixed for a period of time to cover the inside of the wall of the mixing vessel,
b) then, the fine lactose, the magnesium stearate and vilanterol triphenylacetate are completely added onto the first fraction of the lactose in step a) and they are mixed for a period of time (Premix A),
c) second, third and fourth fraction of the coarse lactose are added onto the Premix A respectively (Premix B); after every addition of one fraction of the coarse lactose to the mixture, they are mixed for a period of time,
d) finally, last fraction of the coarse lactose is added onto the Premix B and they are mixed for a period of time to obtain the dry powder formulation of the invention.

9. The dry powder formulation for inhalation according to any one of the claims 1 to 7, wherein vilanterol or a pharmaceutically acceptable salt is in combination with one or more other therapeutic agents that is selected from a group comprising an anti-inflammatory agent, an anticholinergic agent, another β2- adrenoreceptor agonist, an antiinfective agent, or an antihistamine for the preparation of the dry powder formulation.

10. The dry powder formulation for inhalation according to claims 1-7 for use in the treatment or prophylaxis or inflammatory or respiratory tract diseases, wherein vilanterol or a pharmaceutical acceptable salt is in combination with one or more other therapeutic agents that is selected from a group comprising an anti-inflammatory agent, an anticholinergic agent, another beta2-adrenoreceptor agonist, an antiinfective agent, or an antihistamine and wherein
vilanterol or a pharmaceutically acceptable salt and one or more other therapeutic agent(s) may be administered separately, sequentially or simultaneously in separate or combined pharmaceutical formulations in dry powder form.

11. A pharmaceutical product comprising:
- the dry powder formulation for inhalation according to any one of the claims 1 to 7,
- a dry powder formulation for inhalation comprising 6α, 9α-difluoro- 17α-[(2-furanylcarbonyl)oxy]-11β-hydroxy-16α-methyl-3-oxo-androsta-1,4-diene-17β-carbothioic acid S-fluoromethyl ester (fluticasone furoate) and pharmaceutically acceptable carrier and/or additive.

## Patentansprüche

1. Trockenpulver-Formulierung zur Inhalation, umfassend:
- Vilanterol oder ein pharmazeutisch verträgliches Salz davon,
- einen Grobträgerstoff,
- einen Feinträgerstoff, und
- Magnesiumstearat,
**dadurch gekennzeichnet, dass** das Verhältnis des Volumen-Median-Durchmessers des Feinträgerstoffs zum Volumen-Median-Durchmesser des Magnesiumstearats 1:1 beträgt und der Volumen-Median-Durchmesser des Magnesiumstearats zwischen 1µm und 120µm liegt.

2. Trockenpulver-Formulierung zur Inhalation nach Anspruch 1, wobei das pharmazeutisch verträgliche Salz von Vilanteriol Triphenylacetat-Salz ist.

3. Trockenpulver-Formulierung zur Inhalation nach Anspruch 1, wobei der Feinträgerstoff und der Grobträgerstoff aus der Gruppe ausgewählt ist, die Lactose, Mannit, Glucose, Trehalose, Cellobiose, Sorbit, Maltit oder eine Kombination von zwei oder mehreren davon umfasst.

4. Trockenpulver-Formulierung zur Inhalation nach Anspruch 3, wobei der Typ des Feinträgerstoffs gleich oder verschieden vom Typ des Grobträgerstoffs ist.

5. Trockenpulver-Formulierung zur Inhalation nach Anspruch 1, wobei sowohl der Feinträgerstoff als auch der Grobträgerstoff Lactose ist.

6. Trockenpulver-Formulierung zur Inhalation nach einem der vorangehenden Ansprüche, wobei die Menge des Magnesiumstearats weniger als 1,0 Gew.-%, bezogen auf die Gesamtmenge der Trockenpulverformulierung, beträgt.

7. Trockenpulver-Formulierung zur Inhalation nach einem der vorangehenden Ansprüche, wobei die Menge der Feinträgerpartikel weniger als 25 Gew.-%, bezogen auf die Gesamtmenge der Trockenpulverformulierung beträgt.

8. Verfahren zur Herstellung der Trockenpulver-Formulierung zur Inhalation nach einem der vorangegangenen Ansprüche, folgende Schritte aufweisend:
a) die Gesamtmenge der groben Lactose wird in fünf Fraktionen aufgeteilt und die erste Fraktion der groben Lactose wird in ein Mischbehälter gegeben und für einen bestimmten Zeitraum gemischt, so dass die Innenseite der Wand des Mischbehälters abgedeckt ist,
b) anschließend werden die feine Lactose, das Magnesiumstearat und das Vilanteroltriphenylacetat vollständig auf die erste Fraktion der Laktose in Schritt a) gegeben und für einen bestimmten Zeitraum gemischt (Vormischung A),
c) die zweite, dritte und vierte Fraktion der groben Laktose wird der Vormischung A bzw. (Vormischung B) zugegeben; nach jeder Zugabe einer Fraktion der Groblactose zu der Mischung wird diese für eine Zeitdauer gemischt,
d) schließlich wird die letzte Fraktion der Groblactose auf die Vormischung B gegeben und diese für einen bestimmten Zeitraum gemischt, um die Trockenpulverformulierung nach der Erfindung zu erhalten.

9. Trockenpulver-Formulierung zur Inhalation nach einem der Ansprüche 1 bis 7, wobei Vilanterol oder ein pharmazeutisch verträgliches Salz in Kombination mit einem oder mehreren anderen therapeutischen Mitteln verwendet wird, die aus einer Gruppe ausgewählt sind, die ein entzündungshemmendes Mittel umfasst, ein Anticholinergikum, ein anderer Beta₂-Adrenorezeptoragonist, ein antiinfektiöses Mittel oder ein Antihistaminikum zur Herstellung der Trockenpulverformulierung.

10. Trockenpulver-Formulierung zur Inhalation nach einem der Ansprüche 1 bis 7, zur Verwendung bei der Behandlung oder Prophylaxe von entzündlichen Atemwegserkrankungen, wobei Vilanterol oder ein pharmazeutisch verträgliches Salz im Verbund mit einem oder mehreren anderen therapeutischen Mitteln steht, die ausgewählt sind aus einer Gruppe umfassend ein entzündungshemmendes Mittel, ein anticholinerges Mittel, einen anderen Beta₂-Adrenorezeptoragonist, ein antiinfektiöses Mittel oder ein Antihistaminikum, und
wobei Vilanterol oder ein pharmazeutisch verträgliches Salz und ein oder mehrere andere therapeutische Mittel getrennt, aufeinanderfolgend oder gleichzeitig in separaten oder kombinierten pharmazeutischen Formulierungen in Trockenpulverform verabreicht werden können.

11. Pharmazeutisches Produkt, umfassend:
die Trockenpulver-Formulierung zur Inhalation nach einem der Ansprüche 1 bis 7,
eine Trockenpulver-Formulierung zur Inhalation umfassend: 6 a, 9 α-difluor- 17 α-[[2-fluranylcarbonyl)oxy]-11β-hydroxy-16 α-methyl-3-oxo-androsta-1,4-diene-17β-thiocarbonsäure S-fluormethyl ester (Fluticasonfuroat) und einen pharmazeutisch verträglicher Träger und/oder Zusatzstoff.

## Revendications

1. Formulation de poudre sèche pour inhalation comprenant :
- du vilantérol ou un sel pharmaceutiquement acceptable de celui-ci,
- un véhicule grossier,
- un véhicule fin, et
- du stéarate de magnésium,
**caractérisée en ce que** le rapport du diamètre médian en volume du véhicule fin au diamètre médian en volume du stéarate de magnésium est 1:1 et le diamètre médian en volume du stéarate de magnésium est compris entre 1 µm et 120 µm.

2. Formulation de poudre sèche pour inhalation selon la revendication 1, dans laquelle le sel pharmaceutiquement acceptable de vilantérol est le sel triphényl-acétate.

3. Formulation de poudre sèche pour inhalation selon la revendication 1, dans laquelle le véhicule fin et le véhicule grossier sont choisis dans l'ensemble comprenant le lactose, le mannitol, le glucose, le tréhalose, le cellobiose, le sorbitol, le maltitol ou une association de deux ou plus de deux de ceux-ci.

4. Formulation de poudre sèche pour inhalation selon la revendication 3, dans laquelle le type du véhicule fin est identique au ou différent du type du véhicule grossier.

5. Formulation de poudre sèche pour inhalation selon la revendication 4, dans laquelle l'un et l'autre du véhicule fin et du véhicule grossier consistent en lactose.

6. Formulation de poudre sèche pour inhalation selon la revendication 1, dans laquelle la quantité du stéarate de magnésium est inférieure à 1,0 % en poids par rapport à la quantité totale de la formulation de poudre sèche.

7. Formulation de poudre sèche pour inhalation selon l'une quelconque des revendications précédentes, dans laquelle la quantité des particules de véhicule fin est inférieure à 25 % en poids par rapport à la quantité totale de la formulation de poudre sèche.

8. Procédé pour la préparation de la formulation de poudre sèche pour inhalation selon l'une quelconque des revendications précédentes, comprenant les étapes consistant à :
a) diviser en cinq fractions la quantité totale du lactose grossier et introduire dans un récipient de mélange la première fraction du lactose grossier et la mélanger pendant un espace de temps afin de couvrir l'intérieur de la paroi du récipient de mélange,
b) ensuite, ajouter en totalité le lactose fin, le stéarate de magnésium et le triphényl-acétate de vilantérol à la première fraction du lactose dans l'étape a) et les mélanger pendant un espace de temps (Prémélange A),
c) ajouter la deuxième, la troisième et la quatrième fraction du lactose grossier au Prémélange A respectivement (Prémélange B) ; après chaque addition d'une fraction du lactose grossier au mélange, les mélanger pendant un espace de temps,
d) enfin, ajouter la dernière fraction du lactose grossier au Prémélange B et les mélanger pendant un espace de temps pour obtenir la formulation de poudre sèche de l'invention.

9. Formulation de poudre sèche pour inhalation selon l'une quelconque des revendications 1 à 7, dans laquelle le vilantérol ou un sel pharmaceutiquement acceptable est en association avec un ou plusieurs autre(s) agent(s) thérapeutique(s) qui est/sont choisi(s) dans un ensemble comprenant un agent anti-inflammatoire, un agent anticholinergique, un autre agoniste de récepteur adrénergique β2, un agent anti-infectieux, ou un agent antihistaminique, pour la préparation de la formulation de poudre sèche.

10. Formulation de poudre sèche pour inhalation selon les revendications 1 à 7, destinée à être utilisée dans le traitement ou la prophylaxie de maladies inflammatoires ou des voies respiratoires, dans laquelle le vilantérol ou un sel pharmaceutiquement acceptable est en association avec un ou plusieurs autre(s) agent(s) thérapeutique(s) qui est/sont choisi(s) dans un ensemble comprenant un agent anti-inflammatoire, un agent anticholinergique, un autre agoniste de récepteur adrénergique bêta2, un agent anti-infectieux, ou un agent antihistaminique, et dans laquelle le vilantérol ou un sel pharmaceutiquement acceptable et un ou plusieurs autre(s) agent(s) thérapeutique(s) peuvent être administrés séparément, séquentiellement ou simultanément en formulations pharmaceutiques sous forme de poudre sèche séparées ou associées.

11. Produit pharmaceutique comprenant :
- la formulation de poudre sèche pour inhalation selon l'une quelconque des revendications 1 à 7,
- une formulation de poudre sèche pour inhalation comprenant du 6α,9α-difluoro-17α-[(2-furanylcarbonyl)oxy]-11β-hydroxy-16α-méthyl-3-oxo-androsta-1,4-diène-17β-carbothioate de S-fluorométhyle (furoate de fluticasone) et un véhicule et/ou additif pharmaceutiquement acceptable(s).
